(19) Europäisches Patentamt
European Patent Office
Office européen des brevets

(11) Publication number: **0 133 281 A2**

# (12) EUROPEAN PATENT APPLICATION

(21) Application number: **84108757.0**

(22) Date of filing: **24.07.84**

(51) Int. Cl.⁴: **C 08 L 63/00**, C 08 L 63/02, C 08 L 79/08, C 08 G 59/50, C 08 G 59/32, C 08 K 5/18, C 08 K 7/06, C 07 C 147/12, C 07 C 101/62, C 07 D 303/36, C 07 D 207/404

(30) Priority: **01.08.83 US 518872**
**01.08.83 US 518863**
**01.08.83 US 518874**
**01.08.83 US 518873**
**01.08.83 US 518879**
**01.08.83 US 518856**
**01.08.83 US 518875**

(43) Date of publication of application: **20.02.85 Bulletin 85/8**

(84) Designated Contracting States: **BE DE FR GB IT NL SE**

(71) Applicant: **AMERICAN CYANAMID COMPANY, 1937 West Main Street P.O. Box 60, Stamford Connecticut 06904 (US)**

(72) Inventor: **Wang, David Wei, 106 Hoyt Farm Road, New Canaan Connecticut (US)**
Inventor: **Courter, Jeanne Lynn, 35 Benstone Street, Stamford Connecticut (US)**
Inventor: **Kohli, Dalip Kumar, 42 Assisi Way, Norwalk Connecticut (US)**
Inventor: **Hirschbuehler, Kevin, 503 E. Broadway, Bel Air Maryland (US)**
Inventor: **Draney, Daniel Robert, 67 Lincoln Avenue Ext., Norwalk Connecticut (US)**

(74) Representative: **Wächtershäuser, Günter, Dr., Gewürzmühlstrasse 5, D-8000 München 22 (DE)**

## (54) Curable epoxy resin composition.

(57) Curable compositions comprising epoxide prepolymers and polyaminobenzoates, alone, or combined with reinforcements, e.g., graphite fibers, and, optionally modified with second resins. The cured resin fiber matrix compositions exhibit high toughness combined with excellent hot/wet strength. N,N'-dimethyl aromatic diamines are prepared from the corresponding diprimary amines via a two step synthesis forming first the corresponding di-succinylimidomethylamine and then reductively cleaving the succinylimido group. The products are useful, e.g., as epoxy curing agents. N,N,N',N'-tetraglycidyl-1,3-propylene bis (p-aminobenzoate) and homopolymers thereof and their use as curable epoxide prepolymers are also disclosed.

# CURABLE EPOXY RESIN COMPOSITION

## FIELD OF THE INVENTION

This invention relates to improved epoxy resin compositions, curing agents therefor and processes for making and using the same. In addition, it relates to curable epoxy resin compositions comprising reinforcing filaments and epoxy prepolymers combined with aromatic polyamine curing agents.

## BACKGROUND OF THE INVENTION

Epoxy resin compositions are useful to encapsulate electronic components, and as structural adhesives, and the like. Reinforced epoxy resin composites having high strength to weight ratios have found extensive use in the aircraft and aerospace industries, and in other applications where strength, corrosion resistance and light weight are desirable. For instance, fiber resin matix materials have replaced aluminum and other metals in primary and secondary structures of modern military and commercial aircraft. Sporting equipment such as tennis rackets and golf clubs have also adopted fiber resin materials successfully.

Epoxy resin compositions and fiber modifications are abundant. Since the advent of fiber resin matrix materials, much effort has been expended in improving their properties and characteristics, including the development of many different curing systems.

Amine and polyamine curing agents have received wide acceptance, but the toxicity, low solubility, high exotherm and variable curing rates seen with the most commonly used amines, such as m-phenylenediamine, 4,4'-diaminodiphenyl methane and 4,4'-diaminodiphenyl sulfone,

has made further improvement desirable. In particular, for aircraft structural applications, epoxy resins cured with available curing agents are either too brittle or do not have sufficient strength and stiffness under hot/wet conditions. It is disclosed in U.K. Patent 1,182,377, which is incorporated herein by reference, that certain aromatic polyamines are effective as curing agents for a variety of polyepoxides, and the resulting cured compositions are useful as films, moldings, coatings and glass-reinforced laminates. There is no indication in the properties presented in the U.K. Patent that the curing agents exemplified therein will produce the combination of toughness and strength under hot/wet conditions essential for use in the above-mentioned structural applications.

In U.S. 3,932,360, diamine cured polyurethane products are described, in which the diamines are of the formula, e.g.,

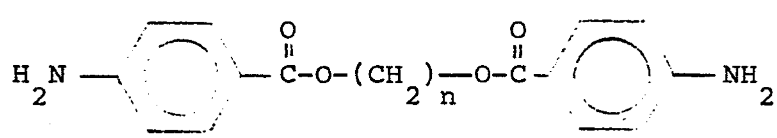

wherein n is an integer from 2 to 12. This '360 patent does not deal with curing compounds having more than one epoxide groups per molecule.

In Gillhan et al., Organic Coatings and Applied Polymer Science Proceedings, Vol. 46, p. 592-598, March-April, 1982, polyepoxides cured with diamines of the immediately preceding formula (n is 3), are described.

The present development relates to curable epoxy resin compositions. In one of its aspects, it provides fiber resin matrixes comprising reinforcing filaments in a heat-curable epoxy resin composition comprising an epoxy prepolymer and a novel family of aromatic polyamine curing agents. No member of this

novel family of curing agents is specifically exemplified in the U.K. Patent. The invention provides neat resin formulations having, after cure, improved physical properties, e.g., higher elongation and satisfactory hot/wet modulus. The epoxy compositions of the present invention, cured with filaments, exhibit improved interlaminar toughness and residual compression strength after impact, while maintaining compression strength under hot/wet conditions.

SUMMARY OF THE INVENTION

According to the present invention improved epoxy resin compositions are provided, particularly as fiber matrix compositions that afford satisfactory compression strength over known matrix formulations, especially under hot/wet conditions, and improved compression strength after impact.

Such compositions comprise:

(a) non-siliceous reinforcing filaments, and

(b) a heat-curable epoxy resin composition comprising:

(i) an epoxy prepolymer or combination of prepolymers having more than one epoxide groups per molecule, and

(ii) an amount effective to promote cure of an amine-functional curing agent or combination of curing agents selected from those of the formula:

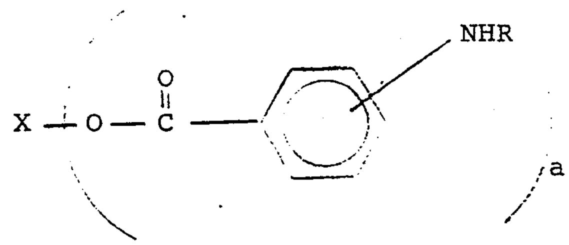

wherein a is 2 or 3, R is hydrogen, alkyl, or aryl, and X is a divalent or trivalent organic hydrocarbon, hetero-interrupted hydrocarbon, or substituted hydrocarbon radical or -N-.

In another aspect, the present invention contemplates fiber reinforced heat-curable epoxy resin compositions comprising:

(i) an epoxy prepolymer or combination of prepolymers having more than one epoxide groups per molecule, and

(ii) an amount effective to promote cure of an amine-functional curing agent or combination of curing agents selected from those of the formula:

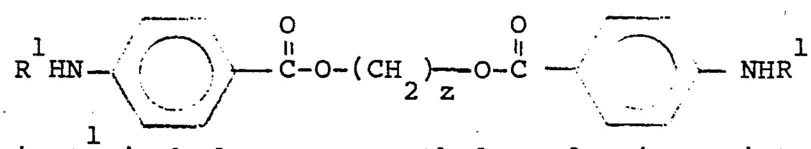

wherein $R^1$ is hydrogen or methyl, and z is an integer of from 2 to 12, preferably 3. Special mention is made of the compound in which $R^1$ is methyl and z is 3.

It is among the features of this aspect of the invention to provide such compositions in filled and/or reinforced, e.g., glass fiber reinforced, embodiments which are useful as prepregs, for example, to make laminates and other structural shapes in accordance with procedures known in this art.

In another preferred feature of the invention, resin fiber matrix compositions will comprise:

(a) reinforcing filaments, and

(b) a heat curable epoxy resin composition formed of the following materials:

(i) N,N,N',N'-tetraglycidyl-4,4'-diaminodiphenyl methane, e.g., 50 to 100, preferably 75 to 85 parts by weight;

(ii) tetraglycidoxy tetraphenylethane, e.g., 0 to 50, preferably, 15 to 25 parts by weight;

(iii) trimethylene bis-(p-aminobenzoate), e.g., 28 to 60, preferably 35 to 45 parts by weight;

(iv) fumed silica, e.g., 0-12, preferably 5 to 7 parts, by weight, and

(v) the reaction product of toluene-diisocyanate and dimethylamine, e.g., 0.1 to 2.5 preferably 0.5 to 1.5 parts by weight.

The fiber resin matrix composition is uniquely suitable for use with an interleaf material to prepare a mechanically superior cured structure.

Still another preferred aspect, the present invention provides compositions of epoxy resins and the above-mentioned diamine curing agents which also include a second resin in an amount sufficient to impart improvements in mechanical properties, especially toughness, while preserving substantial resistance to failure under hot/wet conditions. Such resins can be present homogeneously and also in the form known as interpenetrating polymer networks. Particularly useful in this aspect are resins which include repeating units of the formula:

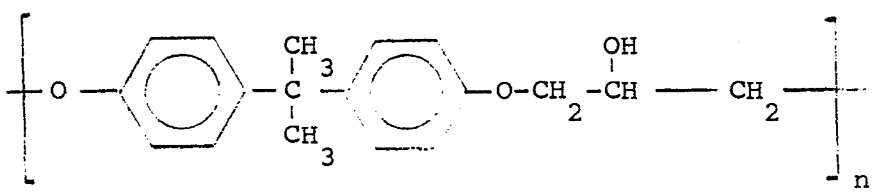

and those with repeating units of the formula:

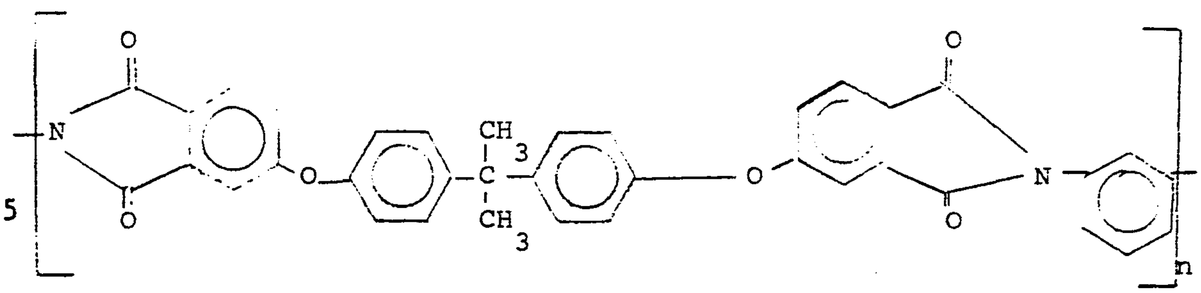

wherein n is a number sufficient to provide a molecular weight of 20,000 to 60,000. Amounts of 5 to 30, preferably 10 to 20 parts by weight per 100 parts by weight of epoxy prepolymer can be used.

This invention also provides a method and intermediates useful in the preparation of N,N'-dimethyl aromatic diamines, to such compounds, and to their use as epoxy curatives, and in other applications.

To this end, there is provided a method for the preparation of a bis-N-methylamino aromatic compound comprising:

(a) reacting the corresponding diprimary amino aromatic compund with succinimide and formaldehyde until formation of the corresponding bis-N-succinimidyl methylamino aromatic compound is substantially complete; and

(b) treating the product of step (a) with sodium borohydride in a solvent until formation of the bis-n-methylamino aromatic compound is substantially complete.

In this aspect, the invention provides compounds of the formula:

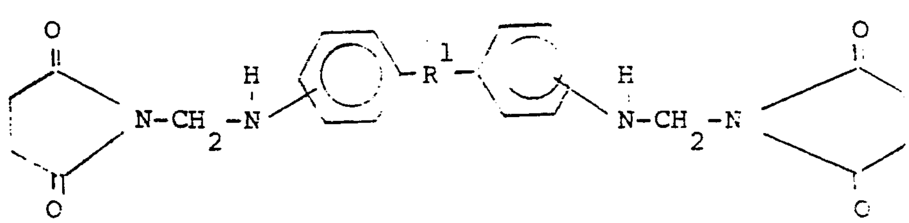

wherein $R^1$ is as above defined. These novel intermediates are useful, for example, to produce N,N'-dimethyl aromatic diamines.

In another feature, the present invention provides compounds of the formula:

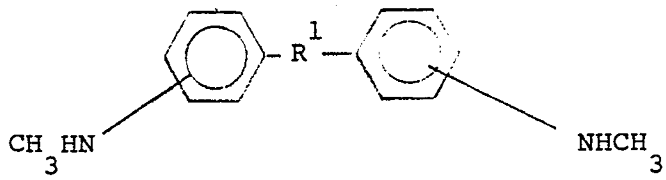

wherein the $CH_3NH-$ groups are in the 4 and 4' positions and $R^1$ is 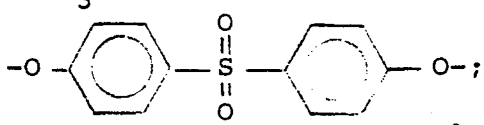; the $CH_3NH-$ groups are in the 3 and 3' positions and $R^1$ is 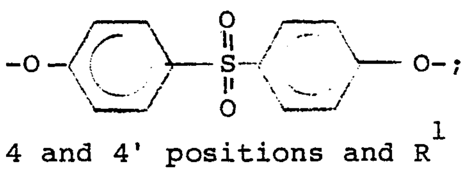; the $CH_3NH-$ groups are in the 4 and 4' positions and $R^1$ is $-\overset{O}{\overset{\|}{C}}O-(CH_2)_n-O-\overset{O}{\overset{\|}{C}}-$ and n is and integer of from 2 to 12; the $CH_3NH-$ groups are in the 2 and 2' positions and $R^1$ is $-SCH_2CH_2S-$; and the $CH_3NH-$ groups are in the 3 and 3' positions and $R^1$ is $-\overset{O}{\overset{\|}{\underset{O}{\underset{\|}{S}}}}-$.

These compounds provide, for example, polyaddition reaction products with polyepoxides having high strength and toughness.

This invention also relates to a new and useful compound. More particularly, it provides N,N,N',N'-tetraglycidyl-1,3-propylene bis(p-aminobenzoate) which is useful as an epoxide resin.

To this end, it has been found that a tetraglycidyl derivative of 1,3-propanediol bis-(p-aminobenzoate), and its homopolymers, can be prepared, that

it has utility, for example, as an epoxy resin prepolymer, and that the polyaddition products thereof with aromatic polyamines have advantageous properties.

DESCRIPTION OF THE DRAWINGS

FIGURE 1 is a schematic of one method for preparing a fiber resin matrix prepreg tape of the present invention.

FIGURE 2 is an enlarged cross-sectional view of a strip of the fiber resin matrix prepreg tape of the invention.

FIGURE 3 is a graphical representation comparing hot/wet compressive strength versus dry impact strength for composites according to this invention with state-of-the-art composites.

DESCRIPTION OF THE PREFERRED EMBODIMENTS

In general, the resin compositions of this invention are prepared by mixing the polyepoxide compounds with the polyamines of the above-mentioned formula in conventional quatitative ratios, e.g., 1 epoxide equivalent to 0.3 to 3.0 NH- equivalents, preferably 1.5 to 2.5 NH- equivalents, optionally with heating, e.g., at a temperature in the range of 30 to 300°C., preferably at a temperature in the range of 80 to 180°C. until a melt is obtained. The melt can then be poured into a mold and reacted, for example, for 2 hours at 135°C. and then for 3 hours at 180°C., to form moldings showing outstanding mechanical and electrical properties. The NH-equivalents is the quantity of aromatic polyamine in grams in which 1 gram - atom of hydrogen combined with amine nitrogen is present.

Fillers, pigments, dyes, reinforcements, such as glass fibers or woven cloths, plasticizers, and mixtures thereof, may be added to the epoxy resin - polyamine composition before the reaction in order to modify ultimate properties, in known ways. Applications can also be made by trowelling, brush coating, immersion or dip-coating, spraying and other convenient method. Catalysts, such as boron trifluoride - organic amine adducts, and the reaction product of toluene 2,4-diisocyanate and dimethylamine can also be included, in quantities of from, e.g., 0.1 to 5% by weight based on the resin - polyamine, to accelerate curing.

The fiber resin matrix compositions according to the present invention can be prepared by embedding filaments, e.g., glass fibers and/or non-siliceous fiaments in a curable resin composition to form a fiber resin matrix which can be manipulated and cured to a solid composite. Particular selection of the filament

material, epoxy prepolymer and curing agent, as well as including optional ingredients such as fillers, dyes, catalysts, processing aids, etc., can give a range of curable compositions heretofore unknown in the art and exhibiting improved physical properties over known materials.

Glass filaments useful herein are well known. The non-siliceous filament component may be of any non-glass, non-silicon dioxide-containing material which improves the strength or other physical properties of the curable epoxy resin component (described infra.). Such filaments include, but are not limited to, filaments comprised of carbon, graphite, silicon carbide, boron, aramid, polyester, polyamide, rayon, polybenzimidazole, polybenzothiazole, metal-coated such filaments, for example nickel-coated and/or silver-coated graphite fibers and filaments, or combinations of such filaments. Fibers (woven or non-woven), tows or mats of such filaments, or tapes (unwoven, flat bundles of the unidirectional filaments) may be employed as desired. In applications demanding high stiffness to weight ratio or shear strength, carbon fibers, graphite filaments, polyaramid filaments or nickel-plated graphite filaments are most preferred.

The epoxy resins suitable for the present invention are compounds having more than one epoxide group per molecule available for reaction with the primary and secondary polyamines of the present invention (described infra.). Such epoxy prepolymers include but are not limited to polyglycidyl ethers of polyvalent phenols, for example pyrocatechol; resorcinol; hydroquinone; 4,4'-dihydroxy-diphenyl methane; 4,4'-dihydroxy-3,3'-dimethyldiphenyl methane; 4,4'-dihydroxydiphenyl dimethyl methane; 4,4'-dihydroxydiphenyl methyl methane; 4,4'-dihydroxydiphenyl cyclohexane; 4,4'-dihydroxy-3,3'-dimethyldiphenyl propane; 4,4'-dihydroxydiphenyl sulphone; or tris-(4-hydroxyphenyl) methane; polyglycidyl ethers of the chlorination and bromination products of the above-mentioned diphenols; polyglycidyl ethers of novolacs (i.e., reaction products of monohydric or polyhydric phenols with aldehydes, formaldehyde in particular, in the presence of acid catalysts); polyglycidyl ethers of diphenols obtained by esterifying 2 mols of the sodium salt of an aromatic hydroxycarboxylic acid with 1 mol. of a dihyalogenoalkane or dihalogen dialkyl ether (U.K. 1,017,612); and polyglycidyl ethers of polyphenols obtained by condensing phenols and long-chain halogen paraffins containing at least 2 halogen atoms (U.K. 1,024,288).

Other suitable compounds include polyepoxy compounds based on aromatic amines and epichlorohydrin, for example N,N'-diglycidyl-aniline; N,N'-dimethyl-N,N'-diglycidyl-4,4'-diaminodiphenyl methane; N,N,N',N'-tetraglycidyl-4,4'-diaminodiphenyl methane; and N-diglycidyl-4-aminophenyl glycidyl ether. Special mention is made of N,N,N',N'-tetraglycidyl-1,3-propylene bis-4-aminobenzoate.

Glycidyl esters and/or epoxycyclohexyl esters of aromatic, aliphatic and cycloaliphatic polycarboxylic acids, for example phthalic acid diglycidyl ester and adipic ester diglycidyl and glycidyl esters of reaction products of 1 mol of an aromatic or cycloaliphatic dicarboxylic acid anhydride and 1/2 mol of a diol or 1/n mol of a polyol with n hydroxyl groups, or hexahydrophthalic acid diglycidyl esters, optionally substituted by methyl groups, are also suitable.

Glycidyl ethers of polyhydric alcohols, for example of 1,4-butanediol; 1,4-butenediol; glycerol; 1,1,1-trimethylol propane; pentaerythritol and polyethylene glycols may also be used. Triglycidyl isocyanurate; and polyglycidyl thioethers of polyvalent thiols, for example of bis mercaptomethylbenzene; and diglycidyltrimethylene sulphone, are also suitable.

Preferably the epoxy prepolymer component will be selected from compounds having the idealized formula:

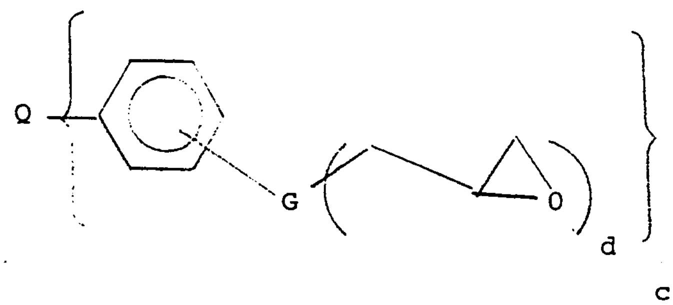

and halogen and alkyl substituted derivatives of such compounds, wherein c is 2, 3 or 4 and equal to the valence of Q; Q is divalent, trivalent or tetravalent radical; G is -O-, NR'- or -N-; R is hydrogen or alkyl; and d is 1 or 2 depending on the valence of G.

The most preferred epoxy compounds will include the following:

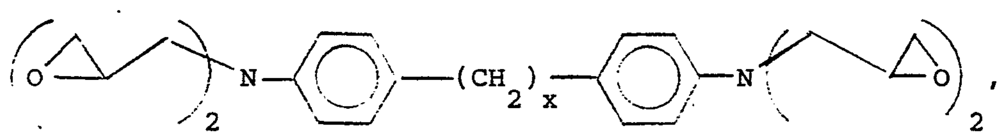

wherein x is an integer from 1 to 4, available commercially (where x=1) as Araldite® MY-720 (Ciba-Geigy);

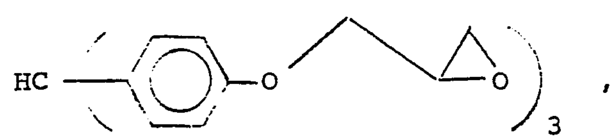

available commercially as XD7342 (Dow Chemical);

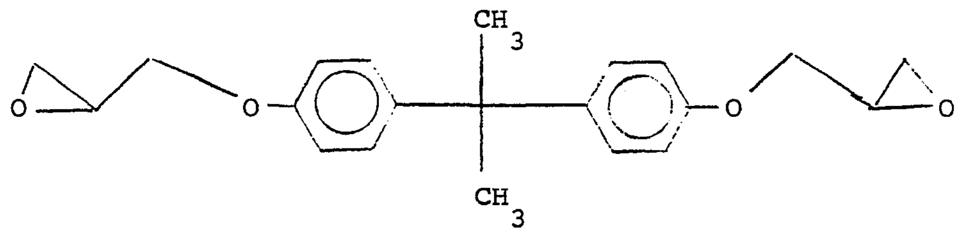

,

available commercially as DER331 (Dow Chemical) or EPON® 828 (Shell);

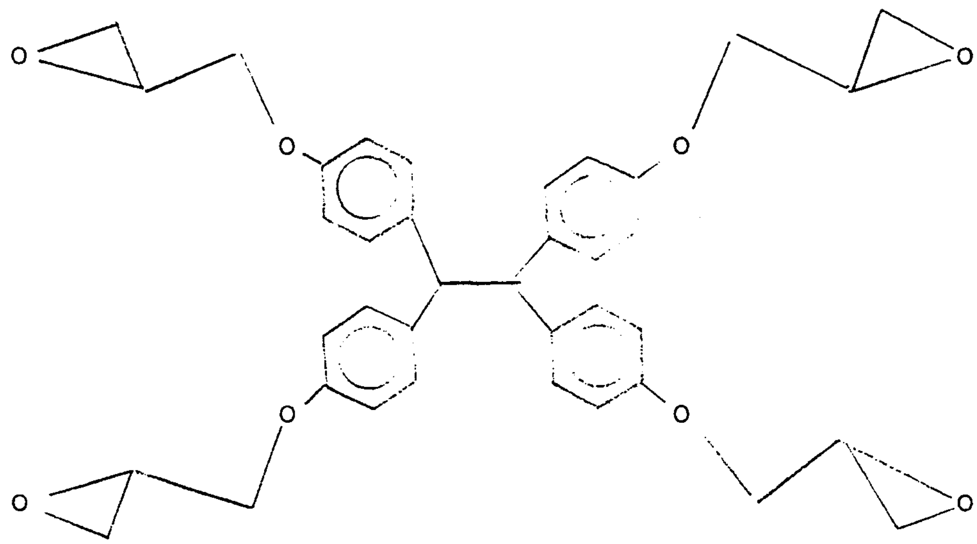

,

available commercially as EPON® 1031 (Shell);

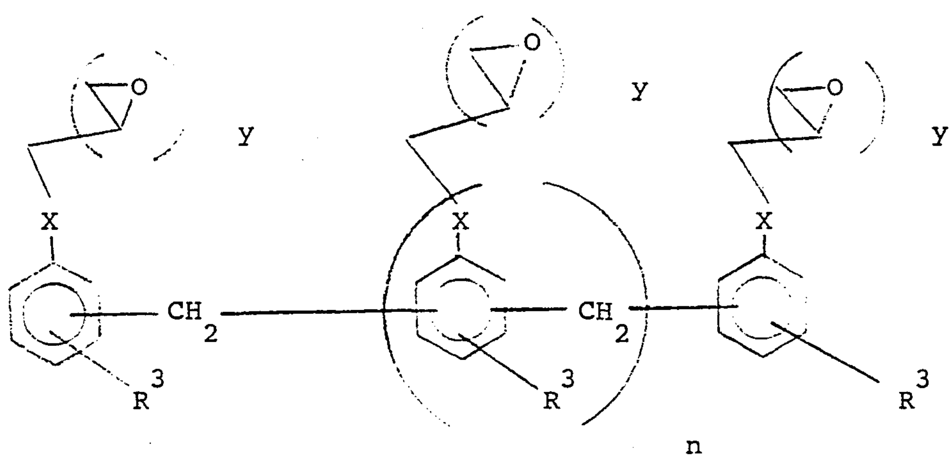

wherein Y is 1 or 2, X is -O- or -N-, $R^3$ is H or $CH_3$ and n is 2 to 8.

Compounds in which X is -O- are available as a mixture under the tradename DEN-438 from Dow Chemical Company.

Also preferred are triglycidyl ethers of meta- and para-hydroxyaniline, e.g., represented by the formula:

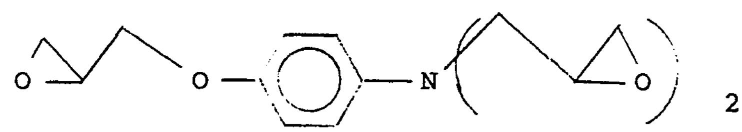

These are available under the tradename ARALDITE® 0500, 0510 from Ciba-Geigy.

The polyamine curing agents are of the formula:

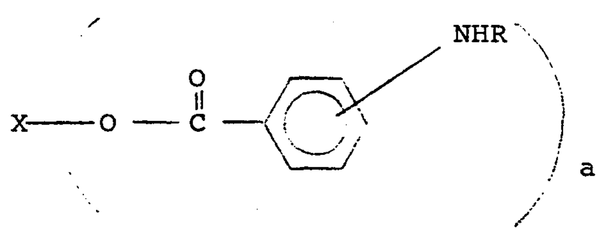

wherein a is 2 or 3, R is hydrogen alkyl or aryl, and X is a divalent or trivalent organic hydrocarbon, hetero-interrupted hydrocarbon, or substituted hydrocarbon radical or -N-. They may be prepared from corresponding starting materials, e.g., nitro compounds, by reduction, for example, according to methods described in U.K. Patent 1,182,377. In addition, the present invention provides an elegant method for N-methylation, using succinimide and formaldehyde with the primary amine, followed by reductive cleavage.

Preferred curing agents are compounds according to the above formula in which R is hydrogen, $C_1$-$C_3$ alkyl, or phenyl and X is a divalent or trivalent radical

of valence a, selected either from (1) a divalent group consisting of $-(CH_2)_y-$, wherein y is an integer of from 2 to 12, $-(CH_2CH_2OCH_2CH_2OCH_2CH_2)-$,

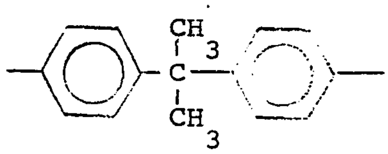

,

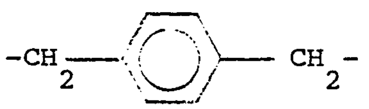

,

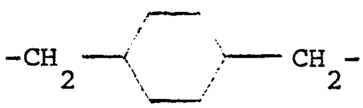

,

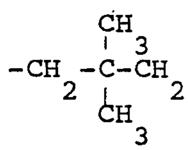

, or (2) a trivalent group consisting of -N- and $-(CH_2)_n-CH-(CH_2)_m-$, wherein n and m are the same or different integers from 1 to 4.

More preferred curing agents are the following:

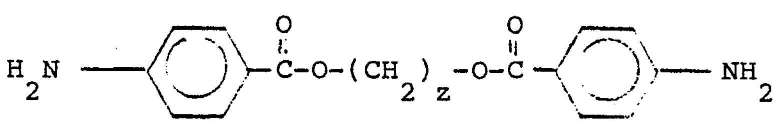

, wherein z is an integer of from 2 to 12, preferably 2 to 6,

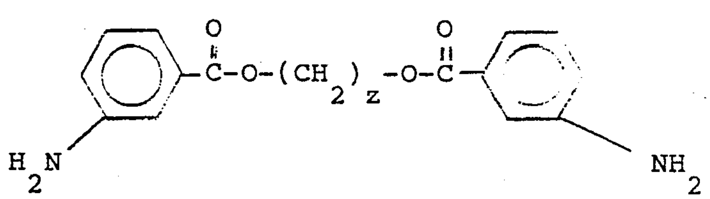

wherein z is an integer from 2 to 12, preferably 2 to 6,

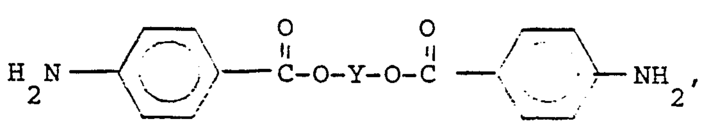

, wherein

Y is $-CH_2CH_2OCH_2CH_2OCH_2CH_2-$,

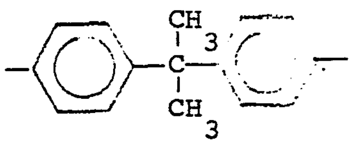 , 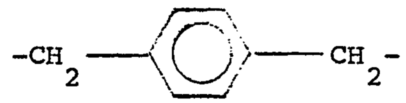 ,

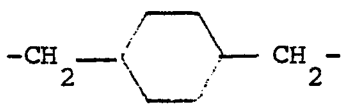 , 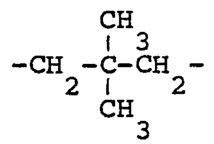 , 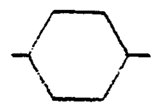 ; or

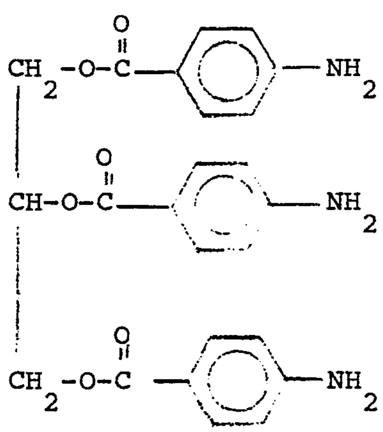 ; 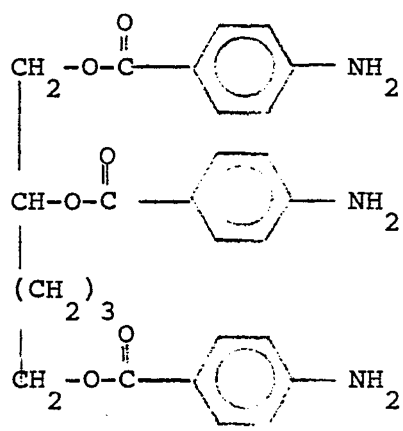 ;

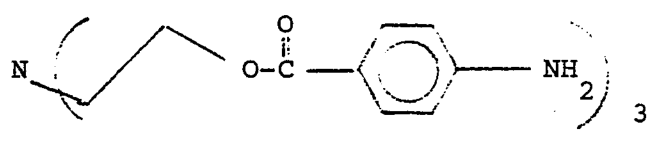 ; and

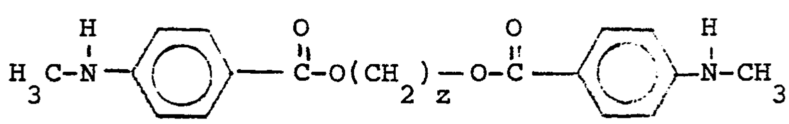, wherein

z is an integer of from 2 to 12, preferably 2 to 6.

In the most preferred compounds, the primary diamine will include one or more of a compound of the formula:

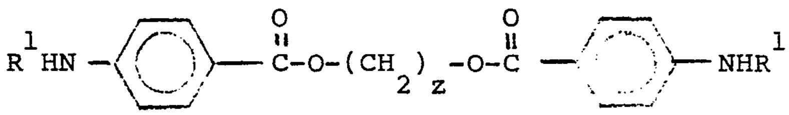

wherein $R^1$ is hydrogen or $C_1$-$C_6$ alkyl, e.g., methyl, and z is an integer of from 2 to 12, preferably 2 to 6, and most preferably 3. Also contemplated are the use of such compounds in combination with other conventional polyamines such as methylene dianiline, phenylene diamine, and the like.

The novel general process of this invention to produce N,N'-dimethyl diamine compounds is broadly applicable to primary aromatic polyamines. These are illustrated above by formula, but preferably group $R^1$ will be

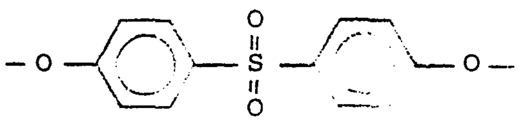 ,

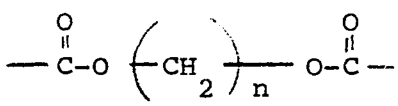 ,

wherein n is 2 to 12,

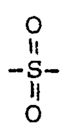 , or

$-S-CH_2CH_2CH_2-S-$.

Also illustrative examples are amines in which $R^1$ is

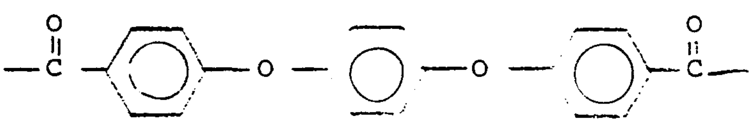 ,

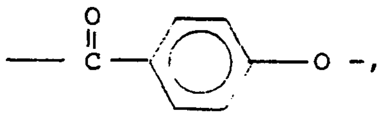 and the like.

Such compounds are either commercially available or they can be readily prepared by those skilled in this art.

The di-primary amines used as certain of the starting materials are also described in the above-mentioned U.S. 3,932,360. In general, they are prepared by reacting p-nitrobenzoyl chloride with a diol and reducing the thus formed compound to a diamine. Illustrative of suitable diols are ethylene glycol, 1,3-propanediol, 1,4-butanediol, 1,5-pentanediol, and 1,6-hexanediol. Preferred is 1,3-propanediol. The preferred diamine starting material is 1,3-propanediol-bis-p-amino benzoate.

As a general procedure, succinimide is reacted with the diamine to produce the corresponding and novel di-succinimidomethyl-substituted diamine. At least a stoichiometric amount of the succinimide will be used. A solvent, such as ethanol, and heating, e.g., at temperatures of from 40°C to 120°C facilitate the reaction. In refluxing ethanol, formation of the disuccinimidomethyl substituted compound is substantially complete in from 2 to 12 hours. The product, which can be recovered by conventional methods, is generally obtained in excellent yield. Typically, yields of greater than 80% of theoretical are obtained. In the next step of the process, the bis-succinimidomethyl compound is treated under conditions which lead to formation of the bis-N-methylamino compound. This is conveniently accomplished by using a combination of a borohydride and a solvent, e.g., an aprotic solvent. Typically, a mixture of sodium borohydride and dimethyl sulfoxide is used. The product is recovered in conventional ways. It is obtained in high yield.

Use of the new compounds as curing agents for epoxy resin is shown hereinafter.

Exemplified hereinafter is the preparation and use of the new N,N,N',N'-tetraglycidyl-1,3-propylene-bis (p-aminobenzoate), and homopolymers thereof, provided by the present invention.

One method of forming the fiber matrix composition of the invention is illustrated in the drawings. As seen in FIGURE 1, the basic fiber matrix material is produced by delivering fiber 2 through conventional eyeboards 4 and 6 to a pressure roller assembly 8. The resin composition is coated in a layer 10 from a conventional film coating applicator 12 onto a substrate such as release paper 14 and passed through the pressure roller assembly 8. Release paper 16 is also delivered to the pressure roller assembly 8.

The pressure rollers 8 are set at a temperature and pressure for imbedding the fibers 2 in the resin layer 10 to form a fiber matrix composition 18. Practice has taught that a temperature in the range of 180°F. and pressures of one thousand pounds over fifteen inch centers are suitable for producing fiber resin prepreg tape 18.

The fibers 2, the substrate 14 with resin layer 10 and the release paper 16 are delivered to the pressure roller 8 and passed therethrough at the rate of 5-20 feet/minute.

The feed of fiber 2 and resin layer 10 to the pressure rollers 8 is selected to produce a fiber matrix of about twenty to sixty weight percent resin and about eighty to forty weight percent fiber. For example, one hundred twenty spools of 6k carbon fibers are delivered within a twelve inch width to the pressure rollers 8 with a layer of resin 0.009 to 0.0013 pounds per square foot. The resulting fiber resin matrix 18 results in a

generally parallel array of fibers, shown by FIGURE 2.

Fillers, pigments, dyes, curing catalysts and other such conventional additives and processing aids may be added to the fiber matrix compositions of the invention before curing to influence the properties of the final resin composite. In addition, polymeric additives such as the butadiene-styrene-acrylonitrile core-shell polymers and the like can be included for their known effects on polymer properties.

The following examples will illustrate the practice of the present invention and are provided by way of demonstration and not by way of limitation.

## EXAMPLES 1-5

The following procedure is used to prepare and cure neat resin compositions: the epoxide prepolymer and the polyamine component are mixed at 135°C for 10 minutes, and cooled to 100°C, the catalyst, if any, is mixed in, and the mixture is degassed for 10 minutes. The liquid resin is then poured into a mold and cured for 2 hours at 135°C and for 3 hours at 180°C. Properties are determined by the following procedures: The flexural test is described in ASTM D-790, Method I. Dynamic mechanical analysis was performed on a Dupont 981 Dynamic Mechanical Analyzer, and $T_g$ was defined as the temperature at which the loss tangent, tan $\delta$, is a maximum. ASTM D4065 test method covers this type of $T_g$ measurement. Conditioning before testing is described by the phrases "wet" and "dry". "Wet" refers to conditioning for two weeks at 71°C, immersing in distilled water, prior to testing at 93°C. "Dry" means testing a sample, as prepared, at 23°C. The formulations tested and the results obtained are set forth in Table 1:

TABLE 1: EPOXY-POLYAMINE CURED COMPOSITIONS AND PROPERTIES

| EXAMPLE | | 1A* | 1 | 2A* | 2 | 3A* | 3 | 4A | 4 | 5 |
|---|---|---|---|---|---|---|---|---|---|---|
| COMPOSITION (equivalents) | | | | | | | | | | |
| N,N,N',N'-tetra-glycidyl 4,4'-diamino diphenyl methane | | 1.0 | 1.0 | 1.0 | 1.0 | 0.88 | 0.88 | 0.88 | 0.88 | 0.10 |
| Tetraglycidoxy tetraphenylethane | | - | - | - | - | 0.12 | 0.12 | 0.12 | 0.12 | - |
| Diaminodiphenyl sulfone | | 0.75 | - | 0.75 | - | 0.77 | 0.77 | 0.75 | - | - |
| Trimethylene bis(p-amino benzoate) | | - | 0.75 | - | 0.75 | - | - | - | 0.75 | 0.25 |
| N,N'-Dimethyl-trimethylene bis-(p-aminobenzoate) | | - | - | - | - | - | - | - | - | 0.50 |
| Reaction product of toluene 2,4-diisocyanate and dimethyl amine (catalyst) | | - | - | 1 (phr) | 1 (phr) | - | - | 1 (phr) | 1 (phr) | |
| PROPERTIES | | | | | | | | | | |
| Modulus, MSI | dry | | 0.51 | 0.54 | 0.50 | 0.61 | 0.53 | 0.54 | 0.52 | 0.50 |
| | wet | 0.42 | 0.36 | 0.39 | 0.29 | - | 0.35 | - | - | 0.34 |
| Strength, KSI | dry | 14.7 | 19.0 | 21.0 | 22.3 | 16.0 | 18.1 | 16.4 | 19.8 | 20.9 |
| Strain, % | dry | 2.6 | 4.0 | 4.1 | 6.1 | 2.6 | 3.6 | 3.0 | 4.3 | 9.2 |
| Work-to-break, in-lbs./in.$^3$ | dry | 210 | 410 | 470 | 850 | 210 | 370 | 270 | 470 | 1575 |
| $T_g$, °C. | dry/wet | 297/- | 252/- | 237/- | 216/- | - | 259/- | - | - | -/176 |

* Control.

The data demonstrate that when the compositions of this invention are cured and tested, in comparison with a standard curing agent, para-diaminodiphenyl sulfone, flexural strength is increased, strain is increased, and work-to-break is increased. Some properties are decreased only slightly. In addition, $T_g$ is reduced by only an average 10%. The advantages of the compositions of this invention are thus shown.

EXAMPLES 6-8

Three fiber resin matrix formulations were prepared from the following materials:

| | |
|---|---|
| component (a) | CELION® 6K high strain graphite fiber |
| component (b)(i) | ARALDITE® MY720 EPON® 1031 (see formulae, supra.) |
| (curing agent) (ii) | trimethylene bis-(p-aminobenzoate) |
| (optional curing agent) | diaminodiphenyl sulfone (DDS) |
| polymer modifier | acrylonitrile-butadiene-styrene, core-shell polymer |
| catalyst | toluene-2,4-diisocyanate reaction product with dimethyl amine |
| filler | fumed colloidal silica (Cab-O-Sil, M-5 Cabot Corp.). |

Using an apparatus shown generally in Fig. 1, prepreg tapes of the structure shown generally in FIg. 2, were prepared:

| EXAMPLE | 6 | 7 | 8 |
|---|---|---|---|
| (28%) Resin mixture (parts by weight) | | | |
| N,N,N',N'-tetra(glycidyl-4,4'-diamino-diphenyl)methane | 80 | 80 | 80 |
| Tetraglycidoxy tetraphenylethane | 20 | 20 | 20 |
| Trimethylene bis-(para-aminobenzoate) | 44 | 44 | 65 |
| Diaminodiphenyl sulfone | -- | -- | 20 |
| Polymer modifier* | -- | 5 | -- |
| Catalyst | 1 | 1 | 1 |
| Fumed silica | 6 | 6 | 6 |

(72%) Filament (parts by weight)
(6K graphite fibers having a strain to failure of about 1.5%)

* BLENDEX 311, Borg-Warner Co.

These samples were cured and compared against commercial epoxy resin matrixes. The sheets of resin involved were as follows:

Uni-Comp : 8 sheets [0]
Quasi-Comp : 16 sheets $[(\pm 45/0/90)_2]$
Comp./Impact : 36 sheets $[(\pm 45/0/90/0/90)_2 - /\pm 45/0/-90/\pm 45]_s$

The compressive strength was measured on a modified ASTM D695 specimen described in D.H. Woolsencraft et al, Composites, Oct., 1981, pages 275-280. Both unidirectional and quasi isotropic laminates were tested by this method. Compressive strength after impact was measured as described in B.A. Byers, NASA Report No. CR 159293, August, 1980. This property is tested by subjecting a cured laminate specimen to 1500 in.-lb. per inch of nominal thickness impact with a 0.62 diameter spherical tip impacter while supported by a rigid base (e.g., 3 x 5 in. steel cutout). The panel is then tested in compression. The results are set forth in Table 2, as follows:

TABLE 2

| EXAMPLE | CONDITION | 8-PLY UNI 23° C | 8-PLY UNI 93° C | 16-PLY QUASI 23° C | 16-PLY QUASI 93° C | COMPRESSIVE STRENGTH AFTER IMPACT 36-PLY (KSI) 1500 in.-lb./in. |
|---|---|---|---|---|---|---|
| 6 | dry* | 189 | 205 | 84 | 93 | 31.5 |
| | wet* | -- | 126 | -- | 71 | |
| 7 | dry | 206 | 178 | 87 | 82 | 32.0 |
| | wet | -- | 130 | -- | 61 | |
| 8 | dry | 205 | 171 | 92 | 74 | 36.0 |
| | wet | -- | 12 to 140 (mean, 34)** | -- | 45 | |
| Commerical No. 1 | dry | -- | -- | -- | -- | 41 |
| Commerical No. 2 | dry | 180 | 175 | 83 | 78 | 28.5 |
| | wet | -- | 145 | -- | 69 | |
| Commerical No. 3 | dry | -- | -- | -- | -- | 20.6 |

* See above.

** For best hot/wet compression strength it would appear that small to moderate excesses of amine are preferred.

Some of the foregoing data are represented graphically also in FIG. 3. The data demonstrate that reinforced compositions according to this invention (Examples 6 and 7) have higher compression strength after impact than two of the three commercial compositions, and better hot/wet compression strength than one of them.

EXAMPLES 9-11

Using the general procedure of Example 1, compositions were prepared and tested. The formulations used, and the results obtained are set forth in Table 3.

TABLE 3: EPOXY/POLYAMINE COMPOSITIONS WITH RESIN ADDITIVES

| EXAMPLE | 9 | 10 | 11 |
|---|---|---|---|
| COMPOSITION (parts by weight) | | | |
| N,N,N',N'-tetraglycidyl-4,4'-diamino diphenyl methane | 100 | 100 | 60 |
| Diglycidyl ether of bisphenol A | - | - | 40 |
| Trimethylene bis(p-aminobenzoate) | 48.4 | 48.4 | 50 |
| Resin modifier A* | 10 | 10 | - |
| Resin modifier B** | - | - | 20 |
| Toluene 2,4-diisocyanate reaction product with dimethylamine (catalyst) | 1 | - | - |
| Boron trifluoride complex with ethylamine (catalyst) | - | 0.5 | - |

*Union Carbide PKHH:

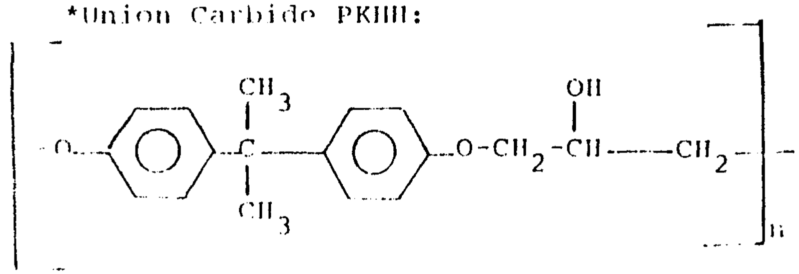

** General Electric ULTEM:

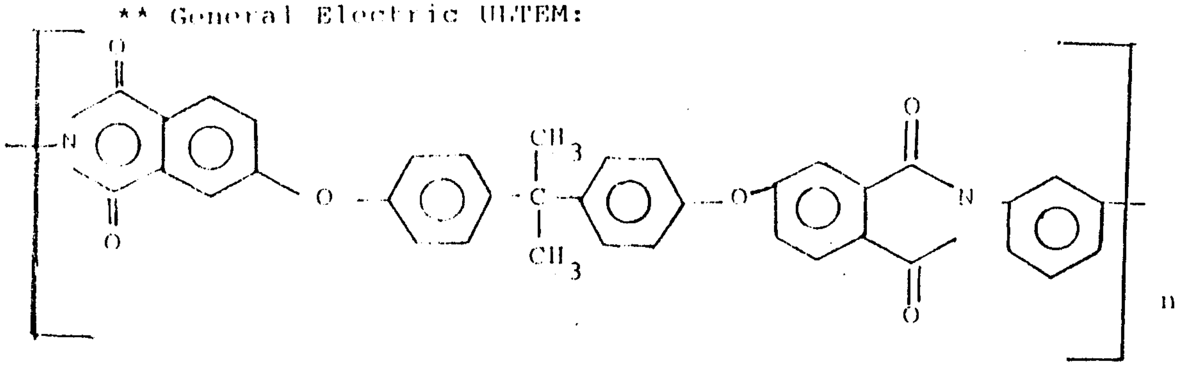

TABLE 3: EPOXY/POLYAMINE COMPOSITIONS WITH RESIN ADDITIVES (continued)

| EXAMPLE | | 9 | 10 | 11 |
|---|---|---|---|---|
| PROPERTIES | | | | |
| Modulus, MSI | dry | 0.53 | 0.55 | 0.50 |
| | wet | 0.33 | 0.28 | 0.36 |
| Strength, KSI | dry | 23.7 | 25.4 | 23.1 |
| Strain, % | dry | 7.9 | 6.3 | 8.1 |
| Work-to-break, in-lbs/in.$^3$, | dry | 1375 | 945 | 1250 |
| Tg, °C. | dry/wet | 193/150 | 220/170 | 205/170 |

EXAMPLES 12-13

By the general procedure of Examples 6-8, the resins of Examples 9 and 10 were made into prepregs with graphite fiber (CELION® high strain graphite fiber). The prepreg had a resin content of 28% and a reinforcement content of 72%, by weight. Thirty six plies were consolidated under heat and pressure into a unidirectional laminate at 150°F. for 1 hour and 350°F. for two hours. Compressive strength after impact was measured 1500 in.-lb./in. thickness, with the following results: Example 12, 34 ksi, and Example 13, 33 ksi., demonstrating excellent properties in this respect.

EXAMPLES 14-17

The general procedure of Example 1 was used to prepare and test compositions according to this invention which also include, methylene dianiline bismaleimide. The formulations used and the results obtained are set forth in Table 4.

TABLE 4: EPOXY COMPOSITIONS AND PROPERTIES

| EXAMPLE | | 14 | 15 | 16 | 17 |
|---|---|---|---|---|---|
| COMPOSITION (parts by weight) | | | | | |
| N,N,N',N'-tetraglycidyl-4,4'-diamino diphenyl methane | | 60 | 60 | 60 | 60 |
| Diglycidyl ether of bisphenol-A | | 40 | 40 | 40 | 40 |
| Trimethylene bis-(p-amino-benzoate | | 50 | 50 | 50 | 50 |
| Methylenedianiline bis maleimide* | | 5 | 10 | 15 | 20 |
| PROPERTIES | | | | | |
| Modulus, MSI | dry | 0.46 | 0.48 | 0.47 | 0.49 |
| Strength, KSI | dry | 23.2 | 21.5 | 22.9 | 23.0 |
| Strain, % | dry | 7.3 | 6.1 | 6.6 | 6.2 |
| Work-to-break, in-lbs./$in.^3$ | dry | 1070 | 810 | 910 | 840 |
| $T_g$, °C. | dry | 207 | 208 | 207 | 206 |

*
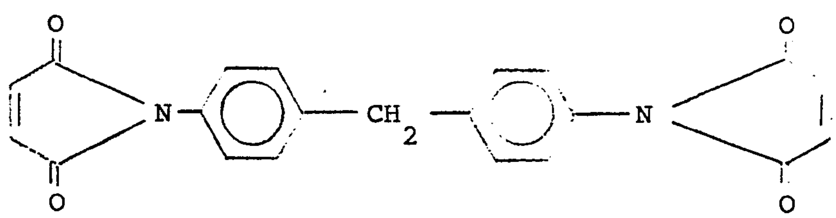

EXAMPLES 18-21

The general procedure of Example 1 was used to prepare and test compositions according to this invention, substituting different epoxy resin prepolymers:

m-TGDDS--

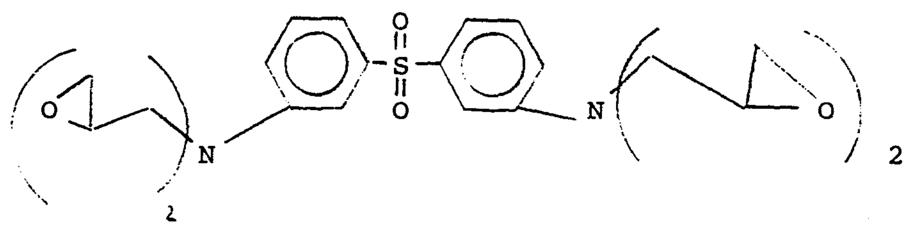

TGPC--

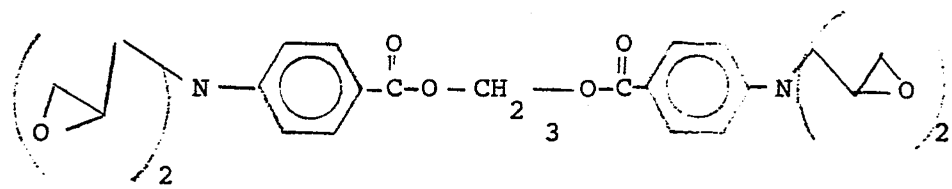

ERL-4299--

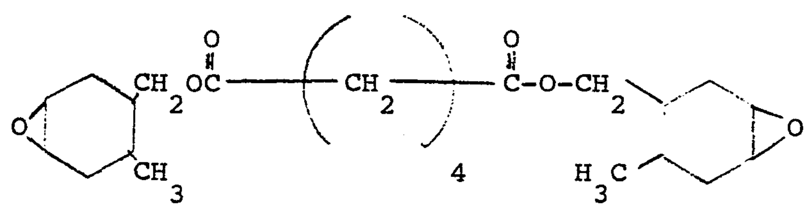

ARALDITE 0510--

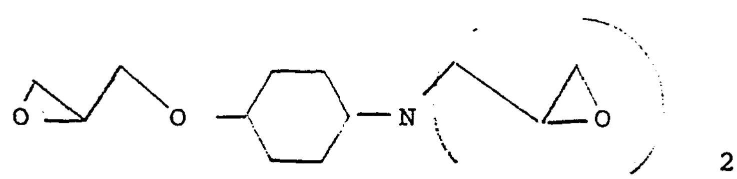

The formulations used and the results obtained are set forth in Table No. 5:

TABLE NO. 5: EPOXY COMPOSITIONS AND PROPERTIES

| EXAMPLE | | 18 | 19 | 20 | 21 |
|---|---|---|---|---|---|
| COMPOSITION (parts by weight) | | | | | |
| N,N,N'N'-tetraglycidyl 3,3'-diamino-diphenyl sulfone | | 100 | -- | -- | -- |
| N,N,N'N'-tetraglycidyl trimethylene bis-(p-aminobenzoate) | | -- | 100 | -- | -- |
| Bis-(3,4-epoxy-6-methylcyclohexylmethyl) adipate | | -- | -- | 100 | 100 |
| N,N-Diglycidyl-4-aminophenyl glycidyl ether | | -- | -- | -- | 100 |
| Trimethylene bis (p-aminobenzoate) | | 51 | 38.5 | 37.4 | 62.8 |
| PROPERTIES | | | | | |
| Modulus, MSI | dry | 0.63 | 0.55 | *NA | 0.53 |
| | wet | 0.35 | 0.18 | NA | 0.26 |
| Strength, KSI | dry | 23.0 | 23.4 | NA | 21.3 |
| Strain, % | dry | 4.0 | 5.5 | NA | 5.6 |
| Work-to-break, in.-lb./$in^3$ | dry | 485 | 770 | NA | 740 |
| Tg, °C. | dry/wet | 240/223 | -- | NA | /156 |

* Not yet available.

## EXAMPLES 22-24

The general procedure of Example 1 was used to prepare and test compositions according to this invention, substituting an N-methylated curing agent. The formulations used and the results obtained are summarized in Table No. 6:

TABLE NO. 6: EPOXY RESIN COMPOSTION AND PROPERTIES

| EXAMPLE | | 22 | 23 | 24 |
|---|---|---|---|---|
| COMPOSITION (equivalents) | | | | |
| N,N,N'N'-tetraglycidyl-4,4'-diamino diphenyl methane | | 1.0 | 1.0 | 1.0 |
| N,N'-dimethyl trimethylene bis-(p-aminobenzoate) | | 1.0 | 0.8 | 0.6 |
| PROPERTIES | | | | |
| Modulus, MSI | dry | 0.49 | 0.49 | 0.48 |
| | wet | 0.19 | 0.22 | 0.25 |
| Strength, KSI | dry | 22.8(y)* | 21.7(y) | 21.9(y) |
| Strain, % | dry | 7.1(y) | 7.1(y) | 7.5(y) |
| Work-to-break, in-lb/in.$^3$ | dry | 1698 | 1600 | 1470 |
| Tg, °C. | dry/wet | 158/120 | 165/- | 163/- |

* (y) = yield

EXAMPLES 25-29

The general procedure of Example 1 was repeated, increasing the ratio of amine equivalents to epoxide equivalents. The formulations used, and the results obtained are shown in Table No. 7:

TABLE NO. 7: INCREASING THE AMINE/EPOXY RATIO

| EXAMPLE | 25 | 26 | 27 | 28 | 29 |
|---|---|---|---|---|---|
| COMPOSITION (equivalents) | | | | | |
| N,N,N',N'-tetraglycidyl-4,4'-diamino diphenyl methane | 1.0 | 1.0 | 1.0 | 1.0 | 1.0 |
| Trimethylene bis(p-aminobenzoate) | 1.0 | 1.25 | 1.5 | 1.75 | 2.0 |
| PROPERTIES | | | | | |
| Modulus, MSI | 0.49 | 0.48 | 0.51 | 0.53 | 0.54 |
| Strength, KSI | 19.0 | 18.8 | 20.4 | 22.7 | 23.1 |
| Strain, % | 4.3 | 4.5 | 4.9 | 5.5 | 5.4 |
| Work-to-break, in-lbs./$in^3$ | 449 | 451 | 560 | 728 | 729 |

The beneficial effect provided by increasing the ratio of amine equivalents to epoxide equivalents is seen from these data.

EXAMPLES 30-35

The general procedures of Example 1 and Examples 25-29 are repeated, including diaminodiphenyl sulfone (DDS) as a co-curing agent and increasing the ratio of the curing agents to epoxide, as was done in Examples 25-29. The formulations used and the results obtained are shown in Table No. 8:

TABLE NO. 8: INCREASING THE AMINE/EPOXY RATIO

| EXAMPLE | 30 | 31 | 32 | 33 | 34 | 35 |
|---|---|---|---|---|---|---|
| COMPOSITION (equivalents) | | | | | | |
| N,N,N',N'-tetraglycidyl-4,4'-diamino diphenyl methane | 1.0 | 1.0 | 1.0 | 1.0 | 1.0 | 1.0 |
| Diaminodiphenyl sulfone | 0.5 | 0.5 | 0.5 | 0.5 | 0.5 | 0.5 |
| Trimethylene bis-(p-aminobenzoate) | 0.75 | 1.0 | 1.25 | 1.50 | 1.75 | 2.0 |
| PROPERTIES | | | | | | |
| Modulus, MIS | 0.51 | 0.50 | 0.54 | 0.57 | 0.60 | 0.60 |
| Strength, KSI | 20.8 | 21.0 | 23.0 | 27.3 | 26.7 | 29.9 |
| Strain, % | 4.8 | 5.0 | 5.0 | 6.8 | 5.7 | 7.4 |
| Work to break, in.-lb./in.$^3$ | 545 | 592 | 655 | 1156 | 915 | 1476 |

The beneficial effect on properties resulting from an increase in the ratio of amine equivalents to epoxide equivalents again is demonstrated.

EXAMPLE 36

Bisphenol A diglycidyl ether plus oligomers (EPON® 828, Shell Chemical Co.) was mixed with trimethylene bis (p-aminobenzoate) at a ratio of 1.0 epoxy equivalents to 0.75 amine equivalents (wt. ratio: 94.9 g. to 30.1 g.). The resin was coated onto graphite fiber (CELION® 6K high strain graphite fiber) and cured into unidirectional 8 ply laminates by heating at 350°F. for 2 hours. The interlaminar strain energy release rate was 5.0 in.-lb./in.$^2$.

EXAMPLE 37

Bisphenol A diglycidyl ether and oligomers (DER® 331, Dow Chemical Co.) was mixed with N,N-dimethyl trimethylene-bis(p-aminobenzoate) at a ratio of 1.0 epoxy equivalents to 0.75 NH- amine equivalents (weight ratio: 75.9:52.3 g.). The resin was coated onto graphite fabrix (CELION® 3K70, plain weave) and cured to a 10 ply laminate by heating at 350°F. for 2 hours. Good quality laminates were produced.

EXAMPLE 38

A mixture comprising tris(4-glycidoxyphenyl) diglycidyl methane (80 parts, Dow Chemical XD-7342), bisphenol A diglycidylether (20 parts, Dow Chemical DER® 331), trimethylene bis(p-aminobenzoate), 38 parts, dicyandiamide, 2 parts, and the reaction product of 2,4-toluene diisocyanate and dimethylamine, 2 parts, all by weight, was prepared and applied to CELION® high strain graphite fibers and made into an 8 ply unidirectional laminate. It had a compression strength of 20.9 x $10^3$ psi at 73°F.

EXAMPLE 39

Tris-(4 glycidoxyphenyl) methane (Dow Chemical, XD-7342) was mixed with N,N'-dimethyl-trimethylene bis(p-aminobenzoate) at a ratio of 1.0 epoxy equivalents to 0.75 amine equivalents (weight ratio: 69.8g: 55.2g). The resin was coated onto graphite fabric (CELION® 3K70, plain weave) and cured into a 10-ply laminate, by heating at 350°F. for 2 hours. Good quality laminates were produced.

EXAMPLE 40

An epoxylated novolac (Dow Chemical DEN® 438) was mixed with trimethylene bis-(p-aminobenzoate) at a ratio of 1.0 epoxy equivalent to 0.75 amine equivalents (weight ratio: 78.9:26.1 g). The resin was coated onto graphite fabric (CELION® 3K70, plain weave) and cured into a 10 ply laminate by heating at 350°F. for 2 hours. Good quality laminates were produced.

EXAMPLE 41

The procedure of Example 40 was repeated, substituting for the polyamine, N,N'-dimethyl-trimethylene bis(p-aminobenzoate) (weight ratio : 72.7 g. epoxy : 52.3 g. diamine). Good quality laminates were produced.

EXAMPLE 42

Bisphenol A diglycidyl ether (DER® 331, Dow Chemical Company.) was mixed with 1,3-trimethylene aminobenzoate at a weight ratio of 94.9 epoxide: 30.1 g. diamine. THe resin was coated onto polyaramid satin weave fabric (DuPont KEVLAR® 285K) and cured into a six ply laminate, by heating at 350°F. for 2 hours. Good quality composites were obtained.

EXAMPLE 43

The procedure of Example 42 was repeated, substituting for the polyamine, N,N'-dimethyl trimethylene bis-(p-aminobenzoate) (weight ratio 75.9 g. epoxy : 52.3 g). Good quality compsites were obtained.

EXAMPLE 44

The procedure of Example 42 was repeated, except that the resin mixture was coated onto nickel plated graphite fibers instead of polyaramid cloth. The matrix composition was cured into 1/4" x 10" x 1/8" composite rods by heating at 350°F. for two hours. Good quality composites were obtained.

EXAMPLE 45

The procedure of Example 43 was repeated, except that the resin mixture was coated onto nickel plated graphite fibers instead of polyaramid cloth. The matrix composition as cured into 1/4" x 10" x 1/8" composite rods by heating at 350°F. for hours. Good quality composites were obtained.

EXAMPLE 46

A resin composition is prepared by mixing the following (by weight)

| | | |
|---|---|---|
| (a) | N,N,N',N'-tetraglycidyl-4,4' diamino diphenyl methane | 120 parts |
| (b) | Polyether polyimide resin (General Electric Ultem, Example 11, above) | 15 parts |
| (c) | Trimethylene bis-p-amino-benzoate | 48 parts |
| (d) | Boron trifluoride-ethylamine complex (catalyst) | 0.5 parts |

A prepreg tape is prepared following the general procedure of 6-8, with a 35 to 45 preferably 40% resin/55 to 65, preferably, 60% graphite loading. When this is formed into laminates by the procedure of Examples 6-8, excellent quality composites are produced. Preferred ranges of compositions are (a) 114-126 parts; (b) 14.25-15.75 parts; (c) 45.6-50.4 parts; and (d) 0.475-0.525 parts.

EXAMPLE 47

(a) N,N'-Disuccinimidomethyl-1,3-trimethylene-bis(p-aminobenzoate). - Two moles of succinimide are reacted with one mole of 1,3-propanediol bis-p-aminobenzoate in refluxing ethanol for 6 hours; and the product is recovered by filtration after cooling, m.p. 198-200°C, yield 82% of theoretical.

(b) N,N'-Dimethyl-1,3-trimethylene-bis-(p-aminobenzoate). - The intermediate of step (a) is treated in dimethyl sulfoxide with sodium borohydride at 85°C for 1 hour. The mixture is poured into water, the product precipitates and is recovered by filtration, m.p. 114°C after recrystallization from methylene chloride-cyclohexane mixture, yield 84% of theoretical.

EXAMPLE 48

By the general pocedure of Example 47, step (a), substituting the corresponding diprimary amines, the following di-N-succinimidomethyl amines can be provided:

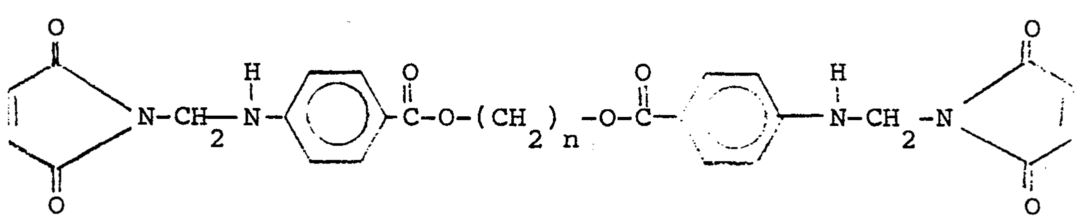

wherein n is, respectively, 2, 4, 5 and 6.

By the general procedure of Example 47, step (b), substituting the corresponding N-succinimidomethyl amines, the following N-methyl amines can be obtained:

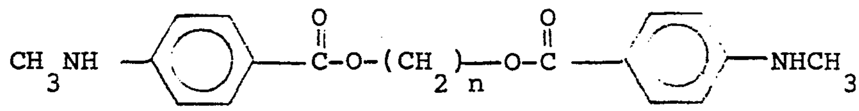

wherein n is, respectively, 2, 4, 5, and 6.

EXAMPLE 49

(a) N,N'-Disuccinimidomethyl-4,4'-bis(p-aminophenoxy) diphenyl sulfone. - The general procedure of Example 47, step (a) is repeated substituting for the bis-aminobenzoate compound, 4,4'-bis(p-aminophenoxy) diphenyl sulfone.

(b) N,N'-Dimethyl-4,4'-bis(p-amino) diphenyl sulfone. - The procedure of Example 47, step (b), was repeated, substituting the intermediate of step (a) herein. The named product, m.p. 73-75°C, was obtained.

EXAMPLE 50

(a) N,N'-Disuccinimidomethyl-4,4'-bis(m-aminophenoxy) diphenyl sulfone. - The procedure of Example 47, step (a) is repeated, substituting 4,4'-bis (m-amino-phenoxy) diphenyl sulfone.

(b) N,N'-Dimethyl-4,4'-bis(m-aminophenoxy) diphenyl sulfone. - The procedure of Example 47, step (b), was repeated, substituting the intermediate of step (a) herein. The named product, m.p., 63-65°C, was obtained.

The products of the examples are mixed with epoxide prepolymers at 135°C for 10 minutes and then degassd under vacuum. The compositions are cured by casting in a mold and cured for two hours at 135°C and then for 3 hours at 180°C. Physical properties are measured by ASTM D-4065. The formulations used and the results obtained are set forth in Table No. 9:

TABLE NO. 9: EPOXY/N-METHYLDIAMINE COMPOSITIONS

| COMPOSITION | | A | B | C | D |
|---|---|---|---|---|---|
| **Formulation (parts by weight)** | | | | | |
| N,N,N',N'-Tetra-glycidyl-4,4-diamino-diphenyl methane | | 100 | 100 | 100 | 100 |
| N,N'-Dimethyl-1,3-trimethylene bis(p-aminobenzoate) (Ex. 1) | | 109 | -- | -- | -- |
| N,N'-Dimethyl-4,4'-bis(p-aminophenoxy) diphenyl sulfone (Ex. 2) | | -- | 92 | 92 | -- |
| N,N'-Dimethyl-4,4'-bis(m-aminophenoxy) diphenyl sulfone (Ex. 3) | | -- | -- | -- | 147 |
| 4,4'-bis(m-aminophenoxy)diphenyl sulfone | | -- | 21.6 | 30 | -- |
| **Properties** | | | | | |
| Modulus, MSI | dry | 0.48 | 0.56 | 0.58 | 0.60 |
| | wet | 0.22 | 0.39 | 0.40 | 0.44 |
| Strength, KSI | dry | 22 | 20.4 | 19.0 | 20.0 |
| Strain, % | dry | 10 | 4.0 | 3.6 | 3.5 |
| Work to break, in-lbs/$in^3$ | | 1600 | 455 | 385 | 385 |
| Tg, °C | dry/wet | 165/137 | 186/162 | 191/159 | 162/134 |

In light of the above description, it is obvious that advantageous results have been obtained with the process of the present invention. The state-of-the-art synthetic methods are limited in scope and utilize a number of steps, whereas the method of this invention is generally applicable and provides high yields in only two steps. By using the bis-methylamino compound of this invention as an epoxy curative, resins can be obtained with improved strength, toughness and hot/wet properties. The new resins can be used for fiber reinforced composites where high strength and toughness are required, for example, in aerospace primary or secondary structures and other similar applications, e.g., automobiles. They can also be used in adhesives.

EXAMPLE 51

1,3-propylene bis(p-aminobenzoate), 314.34 g. (U.S. 3,932,360) is dissolved in a mixture comprising 1560 g. of epichlorohydrin, 500 ml. of ethanol, and 50 ml. of water at about 23°C., then the mixture is heated to 80°C. with stirring for 40 hours. Sodium hydroxide solution, 400 g. of 50% by weight in water, is added dropwise over 45 min. during which the temperature reaches 65°C. The temperature is subsequently maintained at 60-65°C. for 4 hours. The liquid is decanted and the solid is washed with methylene chloride. The combined filtrate and washings are vacuum stripped at 65°C. to leave a viscous residue. This is dissolved in 500 ml. of $CH_2Cl2$, 250 ml. of $H_2O$ and 250 ml. of methyl isobutyl ketone. The organic layer is separated and washed with water (3 X 500 ml.). The solution is dried with anhydrous magnesium sulfate, filtered, and the solvent is vacuum stripped to a final temperature of 130°C. The product, a viscous oil, weighs 497g., 92% of theoretical. Epoxy

equivalent weight is 152 - 153 g./epoxide group. The compound has the formula:

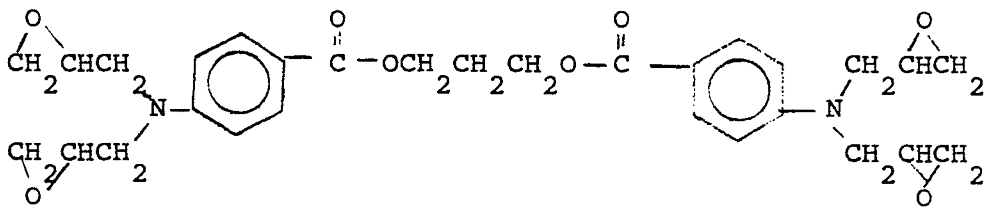

Mixing the compound at a ratio of 1.0 epoxide equivalents with 0.75 NH-amine equivalents of, respectively, diaminodiphenyl sulfone (Composition A) and 1,3-propanediol bis-(p-aminobenzoate) (Composition B) at 110-130°C. and then curing the mixtures at 135-180°C. provided specimens for flexural testing in accordance with ASTM D-790, Method 1. The results were as follows:

| Composition | A | B |
|---|---|---|
| Modulus MSI | 0.58 | 0.57 |
| Strength KSI | 22.7 | 23.4 |
| Strain, % | 4.4 | 5.6 |

The foregoing properties typify advanced composite matrix resins.

The above-mentioned patents, applications and publications are incorporated herein by reference. It is seen that the present invention produces articles of manufacture with beneficial properties, making them useful in a variety of applications. Many variations will suggest themselves to those skilled in this art in light of the foregoing detailed description. All such obvious variations are within the full intended scope of the appended claims.

CLAIMS:

1. A fiber resin matrix composition comprised of:

(a) non-siliceous reinforcing filaments, and

(b) a heat-curable epoxy resin composition comprising:

(i) an epoxy prepolymer or combination of prepolymers having more than one epoxide group per molecule, and

(ii) an amount effective to promote cure of amine functional curing agent or combination of curing agents selected from those of the formula:

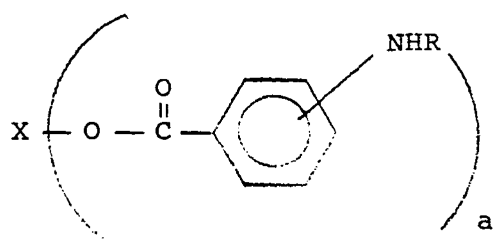

wherein a is 2 or 3, R is hydrogen, alkyl or aryl and X is a divalent or trivalent organic hydrocarbon, hetero-interrupted hydrocarbon, or substituted hydrocarbon radical or -N-.

.

2. A heat-curable epoxy resin composition comprising a reinforcement and

(i) an epoxy prepolymer or combination of prepolymers having more than one epoxide per molecule, and

(ii) an amount effective to promote cure of an amine-functional curing agent or combination of curing agents selected from those of the formula:

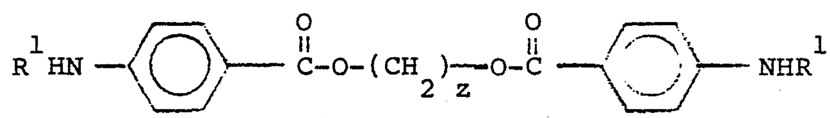

wherein $R^1$ is hydrogen or methyl, and z is an integer of from 2 to 12.

3. A fiber rein matrix composition comprised of:

(a) reinforcing filaments, and

(b) a heat-curable epoxy resin composition formed of the following materials:

(i) N,N,N',N'-tetraglycidyl-4,4-diaminodiphenyl methane,

(ii) tetraglycidoxy tetraphenylethane,

(iii) trimethylene bis-(p-aminobenzoate),

(iv) fumed silica, and

(v) the reaction product of toluene-diisocyanate and dimethylamine.

4. A matrix as in Claim 3 wherein component (b) comprises materials with the following parts by weight allocation:

75-85 parts of (b)(i)
15-24 part of (b)(ii)
35-45 parts of (b)(iii)
5-7 parts of (b)(iv), and
0.5-1.5 parts of (b)(v).

5. A matrix composition as in Claim 1 wherein the resin composition is about 30-40 percent by weight and the filaments are about 70-60 perecent by weight.

6. A matrix composition as in Claim 1 wherein the filaments comprise carbon or graphite filaments.

7. A fiber resin matrix composition comprised of:

(a) non-siliceous reinforcing filaments, and

(b) a heat-curable epoxy resin composition comprising:

(i) an epoxy prepolymer or combination of prepolymers having more than one epoxide group per molecule, and

(ii) an amount effective to promote cure of an amine functional curing agent or combination of curing agents selected from those of the formula:

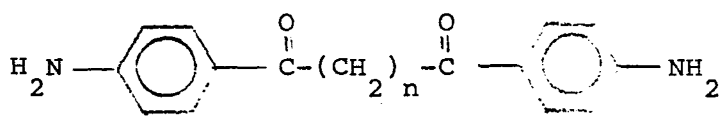

wherein n is an integer of from 2 to 12, the amine-functional curing agent (b)(ii) being present in greater stoichiometric amounts than the epoxy prepolymer (b)(i).

8. A fiber resin matrix composition comprised of:

(a) non-siliceous reinforcing filaments,

(b) a heat-curable epoxy resin composition comprising:

(i) an epoxy prepolymer or combination of prepolymers having more than one epoxide group per molecule,

(ii) an amount effective to promote cure of an amine functional curing agent selected from those of the formula:

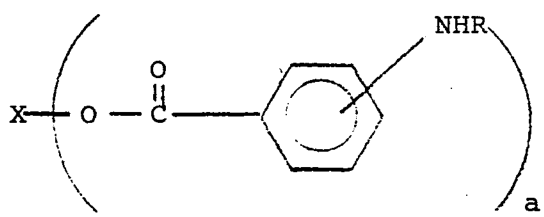

wherein a is 2 or 3, R is hydrogen, alkyl or aryl, and X is divalent or trivalent organic hydrocarbon, hetero-interrupted hydrocarbon, or substituted hydrocarbon radical or -N-, and

(c) a second homogeneous or heterogeneous polyether alcohol or polyetherimide resin component blended and alloyed with composition (b) in an amount sufficient to enhance toughness and resistance to failure under hot/wet stress conditions in composites produced from said composition.

9. A matrix composition as in Claim 8 wherein said second resin (c) comprises a compound of the formula:

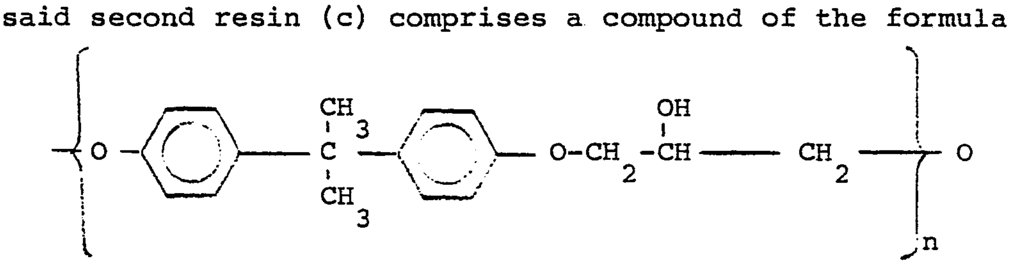

10. A matrix composition as in Claim 8 wherein said second resin (c) comprises a compound of the formula:

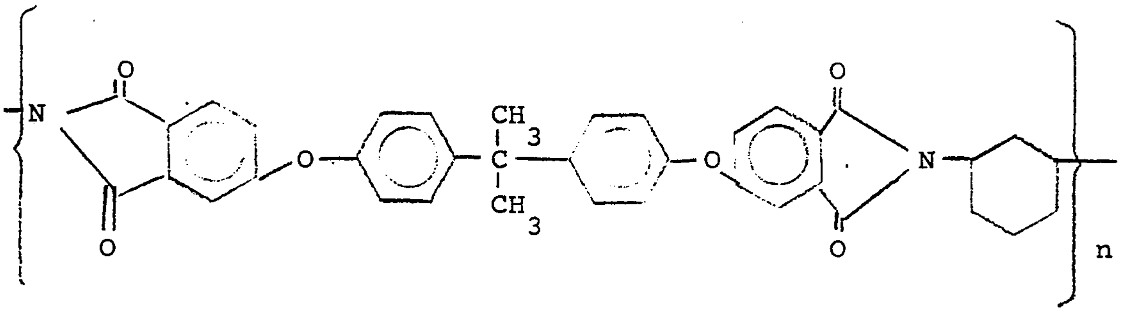

11. A fiber resin matrix composition comprised of:

(a) a reinforcing amount of reinforcing filaments, and

(b) a heat curable epoxy resin composition comprising

(i) 100 parts by weight of N,N,N',N-tetraglycidyl-4,4'-diaminodiphenyl methane;

(ii) 40 to 55 parts by weight of trimethylene bis-(p-aminobenzoate);

(iii) 0.25-4 parts by weight of the reaction product of 2,4-toluene diisocyanate and dimethylamine or boron trifluoride complexed with an organic amine, in combination with

(c) 5 to 20 parts by weight of a resinous reaction product of bisphenol-A and epichlorohydrin of the general formula set forth in Claim 9.

12. A fiber resin matrix composition comprised of:

(a) a reinforcing amount of reinforcing filaments, and

(b) a heat curable epoxy resin composition comprising:

(i) 20 to 90 parts by weight of N,N,N',N'-tetraglycidyl-4,4'-diaminodiphenyl methane

(ii) 10 to 80 parts by weight of the diglycidyl ether of bisphenol-A; and

(iii) 30 to 60 parts by weight of trimethylene bis-(p-aminobenzoate); in combination with

(c) from 10 to 30 parts by weight of a polyether polyimide of the general formula set forth in Claim 10.

13. A fiber resin matrix composition comprised of:

(a) reinforcing filaments, and

(b) a heat curable epoxy resin composition formed from the following materials, in parts by weight:

(i) N,N,N',N'-tetraglycidyl-4,4'-diaminodiphenyl methane, 114-126 parts;

(ii) a polyetherpolyimide resin of the formula:

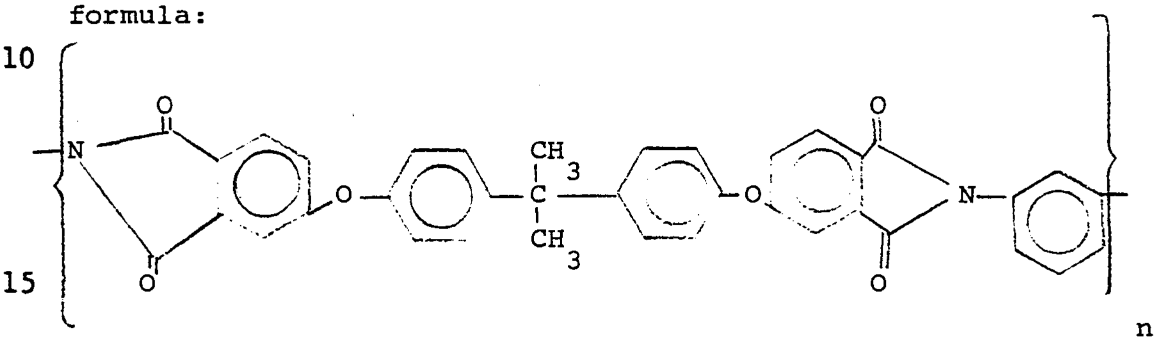

wherein n is an integer sufficient to provide a molecular weight of from 25,000 to 50,000, 14.25 - 15.75 parts;

(iii) 1,3-propylene-bis-(p-aminobenzoate 45.6 - 50.4 parts; and

(iv) boron trifluoride-organic amine complex, 0.475 - 0.525 parts.

14. An article of manufacture comprising a plurality of layers of a fiber-resin matrix composition as defined in Claim 1 consolidated under heat and pressure.

15. N,N,N',N'-tetraglycidyl-1,3-propylene-bis-(p-aminobenzoate), and homopolymers thereof.

16. A method for the preparation of a bis-N-methylamino aromatic compound comprising:

(a) reacting the corresponding diprimary amino aromatic compound with succinimide and formaldehyde to form the corresponding bis-N-succinimidyl methylamino aromatic compound; and

(b) treating the product of step (a) with sodium borohydride in a solvent to form the bis-N-methylamino aromatic compound.

17. A method for the preparation of a bis-N-methyl amino compound of the formula:

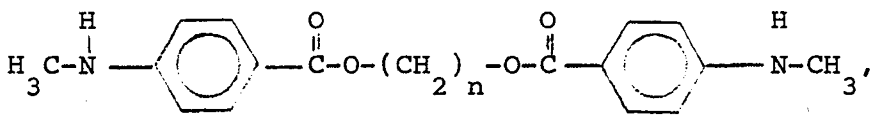

wherein n is an integer of from 2 to 6 comprising

(a) reacting the corresponding diprimary amino compound of the formula:

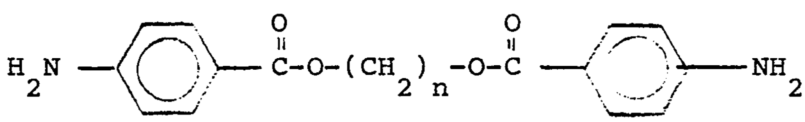

wherein n is as above defined, with succinimide and formaldehyde, until formation of a compound having the formula:

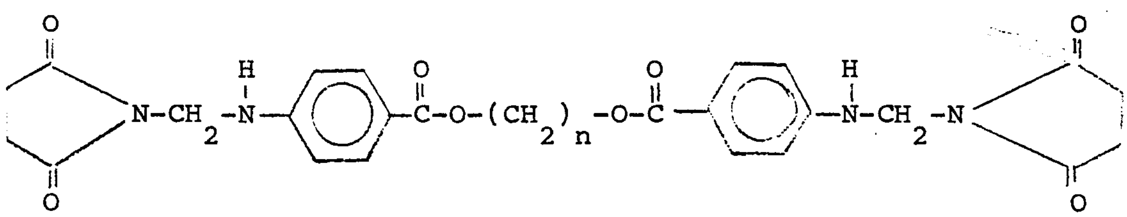

wherein n is as above defined; and

(b) treating said reaction product with sodium borohydride and dimethylsulphoxide until formation of said bis-N-methyl amino compound is substantially complete.

18. A compound of the formula:

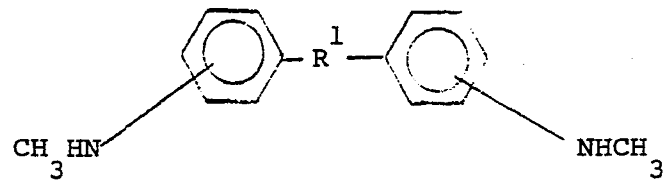

wherein the $CH_3NH-$ groups are in the 4 and 4' positions and $R^1$ is 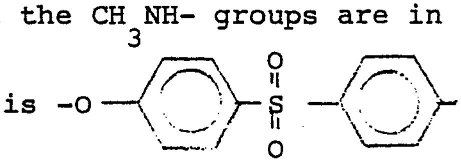; the $CH_3NH-$ groups are in the 3 and 3' positions and $R^1$ is 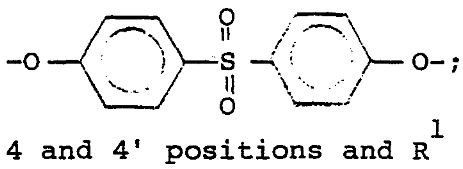; the $CH_3NH-$ groups are in the 4 and 4' positions and $R^1$ is $-\overset{O}{\overset{\|}{C}}O-(CH_2)_n-O-\overset{O}{\overset{\|}{C}}-$ and n is an integer of from 2 to 12; the $CH_3NH-$ groups are in the 2 and 2' positions and $R^1$ is $-SCH_2CH_2S-$; and the $CH_3NH-$ groups are in the 3 and 3' positions and $R^1$ is $-\overset{O}{\overset{\|}{\underset{O}{\underset{\|}{S}}}}-$.

19. A compound of the formula:

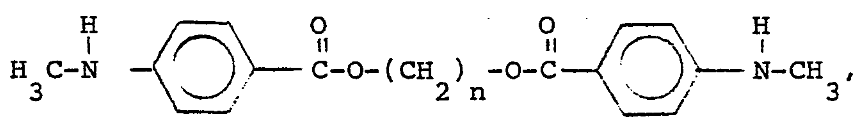

wherein n is an integer of from 2 to 6.

20. A compound of the formula:

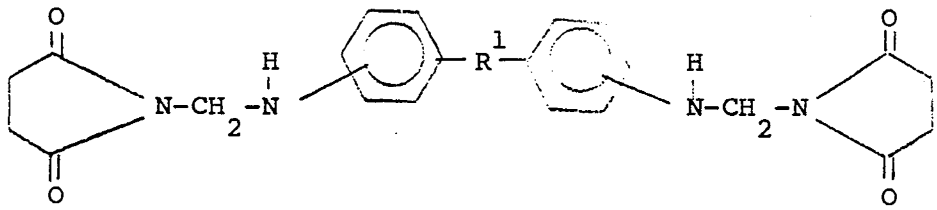

wherein $R^1$ is selected from a direct bond, divalent alkylene of 1 to 20 carbon atoms; divalent alkylene of 1 to 20 carbon atoms terminated, interrupted or terminated and interrupted by -O-, -S-, or -N-; divalent arylene or 6 to 18 carbon atoms; divalent arylene or 6 to 18 carbon atoms terminated, interrupted or terminated and interrupted by divalent alkylene of 1 to 6 carbon atoms, -O-, -S-, -N-, $-\overset{O}{\underset{O}{\overset{\|}{\underset{\|}{S}}}}-$, $-\overset{O}{\overset{\|}{C}}-$, or a combination of any of the foregoing.

21. A compound of the formula:

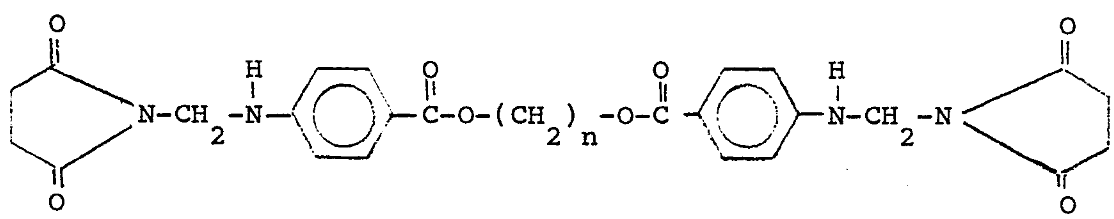

wherein n is an integer of from 2 to 6.

0133281

1/2

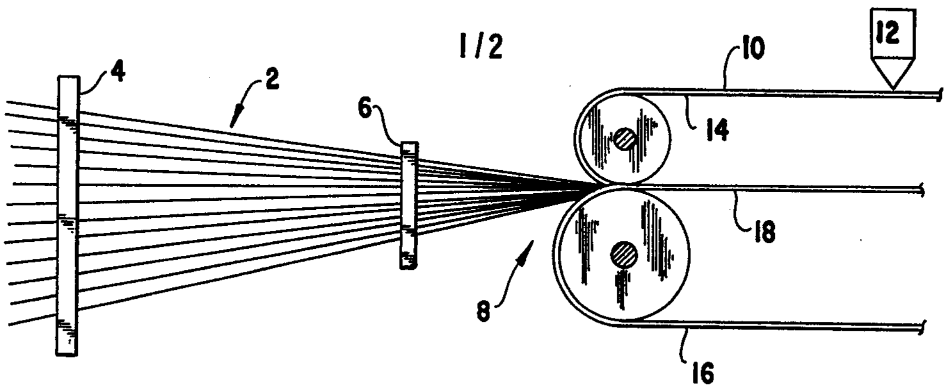


FIG. 1

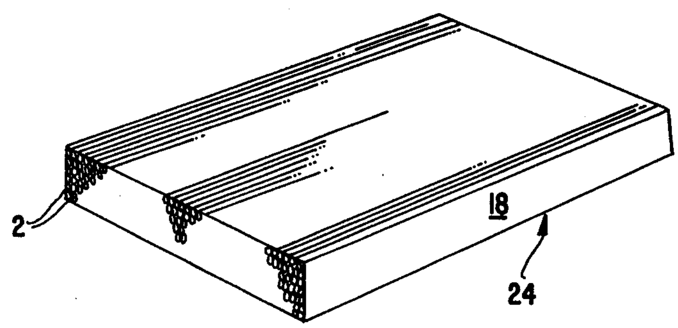


FIG. 2

FIG.3

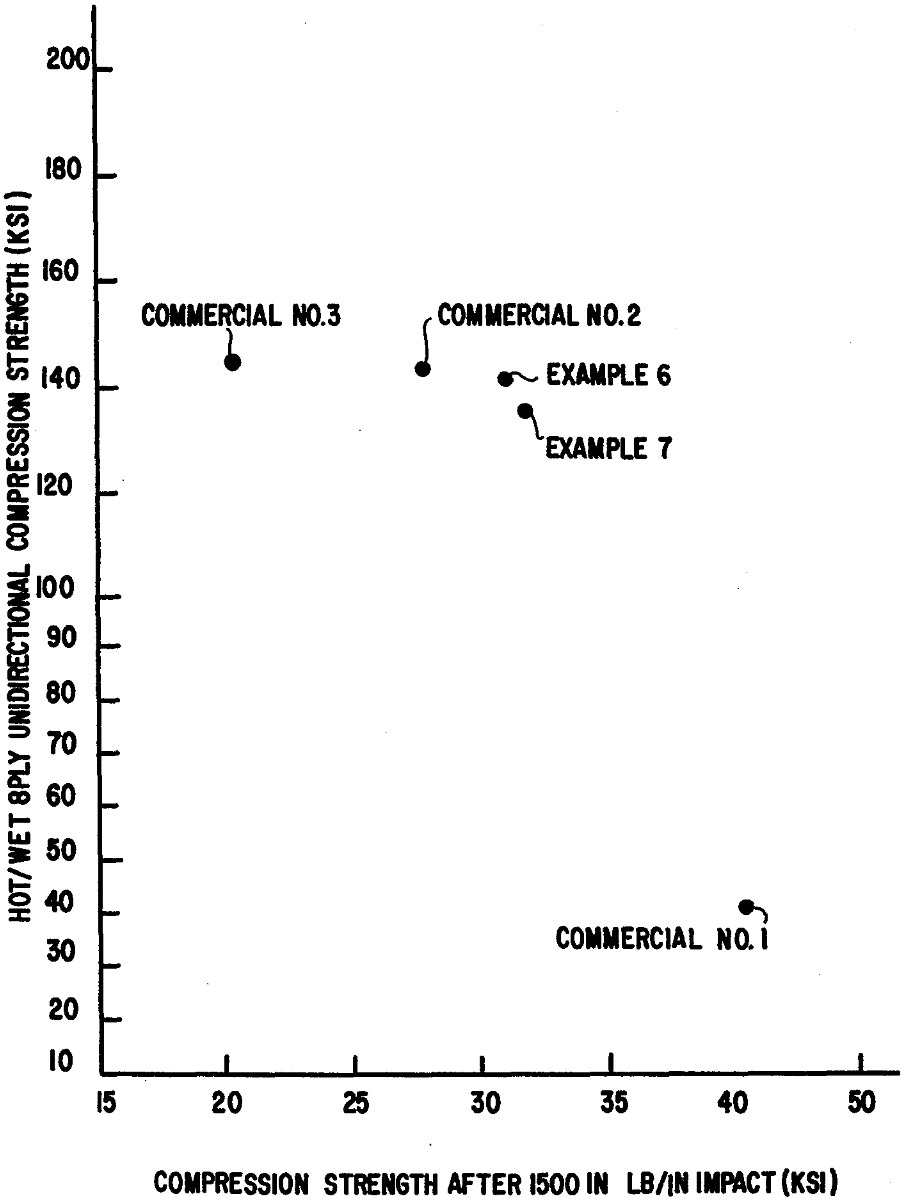
HOT/WET 8PLY UNIDIRECTIONAL COMPRESSION STRENGTH (KSI)
200
180
160
140
120
100
90
80
70
60
50
40
30
20
10
COMMERCIAL NO.3
COMMERCIAL NO.2
EXAMPLE 6
EXAMPLE 7
COMMERCIAL NO.1
15
20
25
30
35
40
50
COMPRESSION STRENGTH AFTER 1500 IN LB/IN IMPACT (KSI)